# EUROPEAN PATENT SPECIFICATION

(11) **EP 4 003 282 B1**
(45) Date of publication and mention of the grant of the patent: **30.08.2023**
(21) Application number: 20746649.1
(22) Date of filing: 29.07.2020
(51) Int. Cl.: A61Q 19/08, A61K 8/37, A61K 8/49, A61Q 19/00, A61K 8/9789, A61K 31/05, A61K 31/202, A61K 36/185, A61K 8/34, A61K 8/36, A61K 8/365

(54) **TREATMENT OF AGING OR UV-DAMAGED SKIN**
BEHANDLUNG VON ALTERNDER ODER UV-GESCHÄDIGTER HAUT
TRAITEMENT DE LA PEAU VIEILLISSANTE OU ENDOMMAGÉE PAR LES UV

(30) Priority: 29.07.2019 EP 19188767; 25.03.2020 EP 20165447
(43) Date of publication of application: 01.06.2022
(73) Proprietor: Echo Pharmaceuticals B.V., 2333 CG Leiden (NL)
(72) Inventor: VERBAKEL, Joost, 2333 CG Leiden (NL); ROZENBLAT, Sharon, 2333 CG Leiden (NL)
(74) Representative: Nederlandsch Octrooibureau
(86) International application number: PCT/EP2020/071342
(87) International publication number: WO 2021/018935

(56) References cited:
- WO-A1-2019/042795
- US-A1- 2015 245 991
- US-A1- 2018 263 952

## Description

### TECHNICAL FIELD OF THE INVENTION

The present invention relates to the treatment of aging or UV-damaged skin, said treatment comprising topically administrating a formulation comprising (i) cannabidiol and (ii) omega-3 fatty acid selected from eicosapentaenoic acid and docosahexaenoic acid and combinations thereof.

The invention further relates to a topical formulation comprising:
- cannabidiol; and
- omega-3 fatty acid selected from eicosapentaenoic acid and docosahexaenoic acid and combinations thereof.

### BACKGROUND OF THE INVENTION

Aging of skin encompasses many physiological effects, which include the skin becoming thinner and becoming more easily damaged. Intensifying this effect is the decreasing ability of skin to heal itself as a person ages. Among other things, skin aging is noted by a decrease in volume and elasticity, as well as increased laxity (sagging) and rhytids (wrinkles).

The process of skin aging is complex and is still not completely understood. There are many known internal and external causes to skin aging. Aging caused by the genes and depending on the passage of time *per se* is called chronological or intrinsic aging. Intrinsic skin aging is characterized by atrophy of the skin with loss of elasticity and slowed metabolic activity. The signs of intrinsic aging are fine wrinkles, thin and transparent skin, loss of underlying fat, facial bone loss, dry skin, inability to sweat sufficiently to cool the skin, hair loss and unwanted hair.

The other type of aging is known as extrinsic aging and is caused by environmental factors. Among harmful environmental factors that contribute to extrinsic aging, long-term effects of repeated exposure to ultraviolet light are most significant and are referred to as photo-aging. It is a cumulative process and depends primarily on the degree of sun exposure and skin pigment. UV irradiation invokes a complex sequence of specific molecular responses that cause damage to the skin connective tissue. Photo-aging affects the sun-exposed areas and is characterized clinically by fine and coarse wrinkling, roughness, dryness, laxity, telangiectasias, loss of tensile strength and pigmentary changes. There is also an increase in development of benign and malignant neoplasms on photo-aged skin.

While inflammatory events, triggered mainly by sun exposure, but also by pollutants, smoking and stress, are the principle cause of rapid extrinsic aging, inflammation also plays a key role in intrinsic aging. Much of the chronological skin aging process that is referred to as intrinsic aging is actually due to epigenetic changes in skin cells, and these changes are due, at least in part, to inflammation.

As time passes, the skin experiences a low level of chronic inflammation that has often been referred to as "smoldering inflammation". This inflammation causes fibroblasts to switch their gene expression pattern from a matrix building phenotype to a matrix destroying one. The level of collagen synthesis decreases while the production of Matrix Metalloproteinases (MMPs) as well as inflammatory cytokines and chemokines increases. Thus, in regard to intrinsic aging, although there is a genetic component to the chronological aging process, it is difficult to separate the long-term aging events that are due purely to genetic factors from those that are caused by years of low level chronic inflammation resulting from oxidative stress, dietary habits, smoking, alcohol consumption, and health.

Although intrinsic skin aging is unavoidable, the process is slow unless the skin is exposed to UV radiation from the sun. For those who spend a lot of time in the sun, the "extrinsic" skin aging process is dramatically accelerated with noticeable photo-damage occurring even at a young age.

A well-known cause of inflammation in both intrinsic and extrinsic aging is the production of Reactive Oxygen Species (ROS). These include the hydroxyl free radical, superoxide radical, nitric oxide radical, and the peroxyl radical. In addition, other chemicals that can be rapidly converted to free radicals and which trigger inflammation, include hydrogen peroxide, hypochlorous acid, and singlet oxygen. In extrinsic aging, exposure of skin to both UVA and UVB radiation causes a rapid increase in NADPH oxidase leading to an increase in ROS, which then activates signalling pathways in keratinocytes in the epidermis and fibroblasts in the dermis, leading to the activation of inflammatory genes. The pathways activated include those that are downstream from the binding and activation of surface receptors including, but not limited to, the EGF receptor, PGE-2 receptor, the TNF-alpha receptor, and IL-1 receptor.

Even without exposure to sunlight or pollutants, as we age, the level of ROS increases in skin cells, caused in part, by genetically programmed cell death, the breakdown of mitochondria, and by lower antioxidant defence capabilities. This increased level of ROS then activates the inflammatory pathways leading to a sustained state of chronic inflammation.

The cytokines and chemokines produced by and secreted from keratinocytes and fibroblasts in the skin in response to UV radiation exposure, further enhance skin aging and inflammation by binding to their specific receptors on adjacent cells in the skin. This binding activates signalling pathways that lead to the further production of inflammatory mediators (a paracrine effect). In addition, cytokines such as IL-1, and TNF-alpha, as well as the prostaglandin, PGE-2, can bind to and activate receptors on the same cells they were produced and secreted from (an autocrine effect). Finally, cytokines such as TNF-alpha, and IL-1, as well as PGE-2 can up-regulate the synthesis of their own receptors, thereby further enhancing the inflammatory response, thus intensifying the damaging effects on the skin.

Production of type I collagen declines during aging, leading to skin thinning and impaired function. Li et al. (Age-associated increase of skin fibroblast-derived prostaglandin E2 contributes to reduced collagen levels in elderly human skin, Invest Dermatol. 2015 September; 135(9): 2181-2188. doi:10.1038/jid.2015.157) report that PGE2 inhibits collagen production by fibroblasts *in vitro* and that PTGES1 and COX2 progressively increase with aging in sun-protected human skin. PTGES1 and COX2 mRNA was increased 3.4-fold and 2.7-fold, respectively, in the dermis of elderly (>80 years) versus young (21-30 years) individuals. Fibroblasts were the major cell source of both enzymes. PGE2 levels were increased 70% in elderly skin. Inhibition of PGE2 synthesis by diclofenac enhanced collagen production in skin organ cultures. The authors conclude that inhibition of PGE2 production may be therapeutically beneficial for combating age-associated collagen deficit in human skin.

It has been suggested in the prior art that skin aging may be slowed down by administering polyphenolic compounds with antioxidant activity or compounds that can block cytokine production.

US 2015/0245991 describes an anti-ageing composition containing cannabidiol, chuanxiong extract, mondo grass extract, Chinese foxglove extract, female and panax ginseng extract, dragon's blood resin, lilyturf root, and jojoba oil.

US 2012/0149775 describes a method for preventing and improving a skin elastic property loss comprising topical administration of a composition containing eicosapentaenoic acid (EPA).

WO 2016/055737 describes cosmetic compositions containing a combination of an extract of the plant *Salvia miltiorrhiza* and niacin and/or niacinamide. These compositions maintain and/or increase the firmness and/or the elasticity of the skin.

US 2002/0098218 describes a method of regulating the condition of mammalian keratinous tissue, said method comprising the step of topically applying a composition comprising one or more phytosterols selected from the group consisting of β-sitosterol, campesterol, brassicasterol, Δ5-avennasterol, lupenol, α-spinasterol, stigmasterol, their derivatives, and combinations thereof.

### SUMMARY OF THE INVENTION

Any references to methods of treatment by therapy in this description are to be interpreted as references to compositions of the present invention for use in those methods.

The present invention provides a treatment of aging or UV-damaged skin comprising topically administrating a formulation comprising (i) cannabidiol and (ii) omega-3 fatty acid selected from eicosapentaenoic acid and docosahexaenoic acid and combinations thereof.

It was unexpectedly found that the aforementioned combination of cannabidiol and omega-3 fatty acid is highly effective in treating aging or UV-damaged skin. Although the inventors do not wish to be bound by theory, it is believed that this high effectiveness is associated with a synergistic interaction between cannabidiol and the omega-3 fatty acid.

The invention further relates to a topical formulation comprising:
- cannabidiol; and,
- omega-3 fatty acid selected from eicosapentaenoic acid and docosahexaenoic acid and combinations thereof.

Cannabidiol, can suitably be provided in the form of plant extracts. Cannabidiol can be extracted from *Cannabis,* the omega-3 fatty acid can be isolated from fish, microalgae, lower fungi, marine bacteria and combinations thereof.

### DETAILED DESCRIPTON OF THE INVENTION

A first aspect of the invention relates to the treatment of aging skin or UV-damaged skin, said treatment comprising topically administrating a formulation comprising (i) cannabidiol and (ii) omega-3 fatty acid selected from eicosapentaenoic acid and docosahexaenoic acid and combinations thereof.

The present treatment preferably comprises topical administration of the formulation to the skin of a human subject. According to a particularly preferred embodiment, the formulation the treatment comprises topical administration to the skin of a human subject. According to a particularly preferred embodiment, the formulation the treatment comprises topical administration to the face of a human subject.

The formulation that is employed in the treatment according to the present invention preferably contains 0.05-3 wt.% cannabidiol, more preferably 0.08-2 wt.% cannabidiol and most preferably 0.1-1 wt.% cannabidiol.

The omega-3 fatty acid selected from eicosapentaenoic acid and docosahexaenoic acid and combinations thereof is preferably present in the formulation in a concentration of 0.0125-3 wt.%, more preferably in a concentration of 0.025-2 wt.% and most preferably in a concentration of 0.05-1 wt.%.

In a preferred embodiment, the formulation contains the omega-3 fatty acid eicosapentaenoic acid. More preferably, the formulation contains at least 0.0125 wt.% eicosapentaenoic acid, even more preferably 0.025-3 wt.% eicosapentaenoic acid and most preferably 0.05-1 wt.% eicosapentaenoic acid.

In another preferred embodiment, the formulation contains the omega-3 fatty acid docosahexaenoic acid. More preferably, the formulation contains at least 0.0125 wt.% docosahexaenoic acid, even more preferably 0.025-2 wt.% docosahexaenoic acid and most preferably 0.05-1 wt.% docosahexaenoic acid.

Most preferably, the omega-3 fatty acid employed in accordance with the present invention is eicosapentaenoic acid.

Cannabidiol and the omega-3 fatty acid are preferably present in the formulation in a weight ratio of 1:10 to 10:1, more preferably in a weight ratio of 1:8 to 8:1.

The inventors have found that the effectiveness of the present formulation can be increased significantly by including one or more phenolic acids selected from danshensu, salvianolic acid A, salvianolic acid B and salvianolic acid C. Accordingly, in a particularly preferred embodiment, the formulation additionally contains one or more phenolic acids selected from danshensu, salvianolic acid A, salvianolic acid B and salvianolic acid C. More preferably, the formulation contains 0.0005-4 wt.% of the one or more phenolic acids, even more preferably 0.002-2 wt.% of the one or more phenolic acids, even more preferably 0.005-1.5 wt.% of the one or more phenolic acids and most preferably 0.01-1 wt.% of the one or more phenolic acids.

The chemical structures of these phenolic acids are shown below

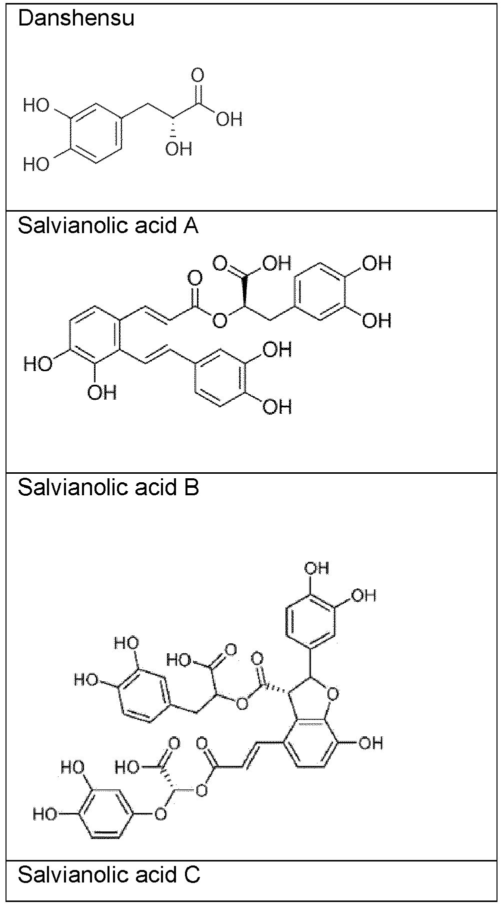

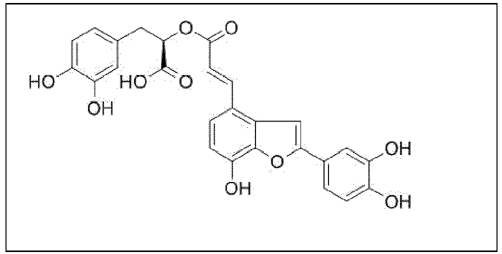

*Salvia miltiorrhiza* is a suitable source of the aforementioned phenolic acids. Since the phenolic acids are water soluble, they can be isolated from *Salvia miltiorrhiza* by aqueous extraction.

In a preferred embodiment, the formulation contains at least 0.0001 wt.% of danshensu, even more preferably 0.0003-0.3 wt.% danshensu and most preferably 0.001-0.1 wt.% danshensu.

The formulation preferably contains one or more salvianolic acids selected from salvianolic acid A, salvianolic acid Band salvianolic acid C in a concentration of 0.0001-3 wt.%, more preferably of 0.003-1 wt. and most preferably of 0.001-0.5 wt.%.

Cannabidiol and the one or more phenolic acids are preferably contained in the formulation in a weight ratio of 80:1 to 1:1, more preferably in a weight ratio of 40:1 to 2:1, most preferably in a weight ratio of 30:1 to 3:1.

The inventors have further found that the effectiveness of the present formulation can also be enhanced by including β-sitosterol. In a preferred embodiment, the composition contains 1-200 mg/kg, more preferably 2-150 mg/kg and most preferably 4-120 mg/kg of β-sitosterol.

Cannabidiol and β-sitosterol are typically contained in the formulation in a weight ratio of cannabidiol: β-sitosterol of 5:1 to 10,000:1, more preferably in a weight ratio of 20:1 to 5,000:1, most preferably in a weight ratio of 50:1 to 2,000:1.

β-Sitosterol is advantageously introduced in the present formulation in the form of hemp seed oil. Besides β-sitosterol, hemp seed oil also contains around 20 wt.% of the omega-3 fatty acid α-linolenic acid. This fatty acid is believed to also contribute to the anti-ageing effect of the present formulation. Typically, besides β-sitosterol, the formulation contains 0.001-0.5 wt.%, more preferably 0.005-0.4 wt.% and most preferably 0.01-0.2 wt.% of α-linolenic acid.

The present formulation preferably comprises a dermatologically acceptable carrier. Examples of dermatologically acceptable carriers include emulsion carriers, including, but not limited to, oil-in-water, water-in-oil, water-in-oil-in-water, and oil-in-water-in-silicone emulsions. Preferred carriers comprise an emulsion such as oil-in-water emulsions and water-in-oil emulsions.

The formulation of the present invention typically comprises 1 to 90 wt.% water. More preferably the formulation comprises 10 to 85 wt.% water, most preferably the formulation comprises 20 to 80 wt.% water.

The present formulation can be provided in different forms. Preferably, the formulation is a gel, a soap, a serum, a cream or a lotion.

A preferred embodiment of the present invention relates to the treatment of UV-damaged skin.

Another preferred embodiment of the present invention relates to the treatment of photo-aging skin. The term "photo-aging" refers to extrinsic aging of skin caused by exposure to UV irradiation.

Another aspect of the invention relates to a topical formulation comprising:
- cannabidiol; and,
- omega-3 fatty acid selected from eicosapentaenoic acid and docosahexaenoic acid and combinations thereof.

Advantageous embodiments of this formulation have already been described above.

Yet another aspect of the invention relates to a method of preparing a topical formulation as described herein before, said method comprising combining an extract of *Cannabis* and an omega-3 oil isolated from a source selected from fish, microalgae, lower fungi, marine bacteria, plant and combinations thereof.

The extract of *Cannabis* that is employed in this method preferably contains at least 20%, more preferably at least 30% and most preferably at least 40% cannabidiol by weight of dry matter.

The omega-3 oil used in the method typically contains at least 30 wt.%, more preferably at least 50 wt.%, more preferably at least 65 wt.% and most preferably at least 80 wt.% of glycerides selected from triglycerides, diglycerides, monoglycerides, phosphoglycerides and combinations thereof.

Omega-3 fatty acids selected from eicospentaenoic acid (EPA), docosahexaenoic acid (DHA) and combinations thereof preferable constitute at least 20 wt.%, even more preferably at least 30 wt.% and most preferably at least 40 wt.% of the fatty acids contained in the omega-3 oil.

In one advantageous embodiment, EPA constitutes at least 20 wt.%, more preferably at least 30 wt.% and most preferably at least 40 wt.% of the fatty acids contained in the omega-3 oil.

In another advantageous embodiment, DHA constitutes at least 20 wt.%, more preferably at least 30 wt.% and most preferably at least 40 wt.% of the fatty acids contained in the omega-3 oil.

The effectiveness of the present formulation for treating aging or UV-damaged skin may be further enhanced by the additional inclusion of an extract of *S*. *miltiorrhiza.* Preferably, the formulation contains 0.01-8 wt.%, more preferably 0.02-4 wt.% and most preferably 0.1-2 wt.% of an extract of *S*. *miltiorrhiza.*

In a preferred embodiment, the extract of *S*. *miltiorrhiza* contains at least 0.5%, more preferably 1-50% and most preferably 5-30% by weight of dry matter, of one or more phenolic acids selected from danshensu, salvianolic acid A, salvianolic acid Band salvianolic acid C.

Preferably, the extract of *S*. *miltiorrhiza* is an extract that has been obtained by extraction with a polar liquid selected from water, methanol, ethanol, iso-propanol and combinations thereof. Most preferably the extract of *S*. *miltiorrhiza* is an aqueous extract.

The effectiveness of the present formulation can also be enhanced by including hemp seed oil. Preferably, the formulation contains 0.001-3 wt., more preferably 0.005-2 wt.% and most preferably 0.01-1 wt.% of hemp seed oil.

The hemp seed oil used in the present method typically contains at least 60 wt.%, more preferably at least 70 wt.% and most preferably at least 75 wt.% of glycerides selected from triglycerides, diglycerides, monoglycerides, phophoglycerides and combinations of

The hemp seed oil typically contains 0.5-5 wt.%, more preferably 1-4 wt.% and most preferably 1.2-3 wt.% β-sitosterol.

Besides β-sitosterol, the hemp seed oil preferably contains 8-40 wt.%, more preferably 10-35 wt.% and most preferably 12-28 wt.% of α-linolenic acid.

The invention is further illustrated by the following non-limiting examples.

### EXAMPLES

### Example 1

The impact of cannabidiol, eicosapentaenoic acid (EPA) and combinations of these components on the UV-B induced secretion of prostaglandin E2 (PGE₂) was investigated in keratinocytes HaCaT cells.

The specifications of the aforementioned components are provided in Table 1.

**Table 1**

| | **Supplier** | **Characterisitcs** |
|---|---|---|
| **Cannabidiol** | Echo Pharmaceuticals BV, the Netherlands | >95% pure extracted CBD |
| **EPA** | KD Pharma, Germany | Acid value 3 mg KOH/g, 67.2 wt.% EPA (as FFA), 0.2% mixed natural tocopherol |

### UV-B induced secretion of PGE₂

This assay was conducted on Human Epidermal Keratinocyte cells (HaCaT) and was carried out in triplicates. After reaching the required confluency, the cells were treated with pre-prepared growth medium containing the test component(s) at a final volume of 200 µL/well. Growth medium without test component was used to prepare the blank samples.

The cells were incubated for 24 hr. Then, the medium was replaced with PBS (containing the test component) and the cells were subjected to 25 mJ/cm² (Table 2) or 12.5 mJ/cm² (Table 3) UV-B irradiation. The flux was determined by an external AccuMAXTM Meter.

Immediately after irradiation, the PBS was aspirated, and the cells were further incubated with freshly prepared media incubated for 24 hr. at 37°C with 5% CO₂. After incubation, the conditioned medium of the different treatment groups was collected under standardized conditions and centrifuged at 250 x g for 5 min to remove particulates. Clear supernatants were frozen at -70°C until analyses.

The UVB-induced cytokines secretion of PGE₂ was measured by commercial ELISA, according to the manufacturer's instructions. The percentage inhibition was determined by comparing PGE₂ levels in the test samples with PGE₂ level in the stimulated controls. The results are shown in Table 2.

**Table 2**

| **Sample** | **Cannabidiol** | **µg/ml EPA** | **Inhibition(%) PGE₂** |
|---|---|---|---|
| 1 | 1.25 | 0 | 0 |
| 2 | 10 | 0 | 17 |
| 3 | 0 | 1.25 | -11 |
| 4 | 0 | 10 | 1 |
| 5 | 1.25 | 1.25 | 21 |
| 6 | 10 | 1.25 | 22 |
| 7 | 10 | 10 | 34 |

### Example 2

Example 1 was repeated in HaCaT cells, using the lower UV-B dosage (12.5 mJ/cm² flux). In addition, an extra test component was used (docosahexaenoic acid). The specification of the latter component is provided in Table 3.

**Table 3**

| | **Supplier** | **Characterisitcs** |
|---|---|---|
| **DHA** | KD Pharma, Germany | Acid value 1.5 mg KOH/g, 72.7 wt.% DHA (as FFA), 0.2% mixed natural tocopherol |

The results of the measurements are summarised in Table 4.

**Table 4**

| **Sample** | **µg/ml** | | | **Inhibition (%)** |
|---|---|---|---|---|
| | **Cannabidiol** | **EPA** | **DHA** | **PGE₂** |
| 1 | 1.25 | 0 | 0 | -11 |
| 2 | 10 | 0 | 0 | -8 |
| 3 | 0 | 1.25 | 0 | -3 |
| 4 | 0 | 10 | 0 | 5 |
| 5 | 0 | 0 | 1.25 | 15 |
| 6 | 1.25 | 1.25 | 0 | 45 |
| 7 | 1.25 | 10 | 0 | 43 |
| 8 | 10 | 1.25 | 0 | 45 |
| 9 | 10 | 0 | 1.25 | 48 |

### Example 3

The impact of cannabidiol, EPA oil and combinations of these components on the UV-B induced secretion of interleukin 8 (IL-8) was investigated in HaCaT cells using the same ingredients and assay as in Example 1, except that this time secretion of IL-8 was determined.

### UV-B induced secretion of IL-8

The percentage inhibition was determined by comparing IL-8 levels in the test samples with the IL-8 level in the stimulated controls. The results are summarised in Table 5.

**Table 5**

| **Sample** | **µg/ml** | | **Inhibition (%)** |
|---|---|---|---|
| | **Cannabidiol** | **EPA** | **IL-8** |
| 1 | 1.25 | 0 | -1 |
| 2 | 10 | 0 | 3 |
| 3 | 0 | 1.25 | -6 |
| 4 | 0 | 10 | -16 |
| 5 | 10 | 1.25 | 24 |
| 6 | 10 | 10 | 22 |

### Example 4

Inhibition of PGE2 was also tested on murine macrophage cell line (RAW 264.7). Beside cannabidiol and EPA, an additional test component was used (extract of S. *miltiorrhiza).* The specification of the latter component is provided in Table 6.

**Table 6**

| | **Supplier** | **Characteristics** | |
|---|---|---|---|
| ***S*. *miltiorrhiza*** | Draco Natural Products, USA | Salvia Powdered extract 1% Danshensu by HPLC. Root extract. | |
| | | | Spray dried, fine powdered botanical extract of ***Salvia miltiorrhiza*** which has been extracted in a lower-temperature process. |
| | | Extraction ratio around 8:1. | |

The results of the measurements are summarised in Table 7.

**Table 7**

| **Sample** | **µg/ml** | | | **Inhibition (%)** |
|---|---|---|---|---|
| | **Cannabidiol** | **EPA** | ***S*. *miltiorrhiza*** | **PGE₂** |
| 1 | 2.5 | 2.5 | 0 | 32 |
| 2 | 2.5 | 2.5 | 2.5 | 49 |

### Example 5

The effect of a combination of cannabidiol and eicosapentaenoic acid (EPA) on the secretion of nitric oxide was determined in macrophage RAW 264.7 cells. The cannabidiol and EPA used were the same as in Example 1. The effect on nitric oxide reduction was also determined for a combination of cannabidiol, EPA and *S*. *miltiorrhiza* extract, and for a combination of cannabidiol, EPA, *S*. *miltiorrhiza* extract and hemp oil. The *S*. *miltiorrhiza* extract used was the same as in Example 4. The specification of the hemp oil is provided in Table 8.

**Table 8**

| | **Supplier** | **Characteristics** |
|---|---|---|
| **Hemp oil** | Vitaprom (senior vital Boca Raton) | Hemp seed oil from *Cannabis Sativa L* (Hemp). |
| | | Contains 18.4% alpha linoleic acid, 3% stearidonic fatty acid and 52.9% linoleic acid |

### Secretion of nitric oxide

RAW 264.7 cells (approx. 2.5×10⁵ ¢/ml, by counting) were seeded in 96 well plates containing 170 µl/well of complete growth medium. The cells were incubated at 37°C with 5% CO₂ for 24 hr. Then, the medium was aspirated and replaced by LPS-containing medium (12.5 ng/ml) without or with the test components. In addition, naive cells, vehicle-treated cells, Stimulated Control and Stimulated Vehicle Control served as negative controls. Dexamethasone served as a positive control for anti-inflammatory assays. A Blank control group was included in the assay.

The cells were incubated at 37°C with 5% CO₂ for 24 hr. At the end of incubation, the viability of the cells was measured using the MTT assay, according to SOP. In addition, the spent media from all test groups were collected under standardized conditions and centrifuged at 250g for 5 min to remove particulates. Clear supernatants were frozen at -70°C until nitric oxide analysis.

The results of the measurements are summarised in Table 9.

**Table 9**

| **Sample** | **µg/ml** | | | | **Inhibition (%)** |
|---|---|---|---|---|---|
| | **Cannabidiol** | **EPA** | ***S. miltiorrhiza*** | **Hemp oil** | **nitric oxide** |
| 1 | 1.25 | 1.25 | 0 | 0 | 13 |
| 2 | 1.25 | 1.25 | 1.25 | 0 | 26 |
| 3 | 1.25 | 1.25 | 1.25 | 0.625 | 39 |

### Example 6

The effect on epidermal viability and the effect on UV-B induced secretion of PGE₂ and IL-8 secretion was determined for a combination of cannabidiol, EPA, *S*. *miltiorrhiza extract* and hemp oil. The specifications of these ingredients were the same as in Examples 1, 4 and 5.

Fixed size skin explant pieces (0.64 cm²) were cut from the skin tissue, using a designated press apparatus. The skin pieces were prepared and maintained in air liquid interphase. The explants were laid in 6-well culture plates containing skin culture medium (DMEM supplemented with 100U/ml penicillin and 100 µg/ml streptomycin), dermal side down in the medium and epidermis phasing up. The pieces were left to recover overnight at 37°C with 5% CO₂.

### Anti-aging evaluation

The assay was carried out in triplicate.

Skin pieces were treated with skin creams having the compositions shown Table 10.

**Table 10**

| | **Skin cream 1** | **Skin cream 2** | **Skin cream 3 (Placebo)** |
|---|---|---|---|
| Cannabidiol | 0.2 | 0.5 | 0 |
| EPA | 0.2 | 0.5 | 0 |
| *S*. *miltiorrhiza* | 0.2 | 0.5 | 0 |
| Hemp oil | 0.1 | 0.25 | 0 |
| Cream base | remainder | | 100.0 |

15 min post application, skin pieces were transferred to PBS and subjected to 400 mJ/cm² UVB irradiation, and then returned to the growth medium. After 24 hr, the spent medium was collected under standardized conditions and centrifuged at 250 x g for 5 min to remove particulates. The supernatants were frozen at -70°C until cytokine analysis.

UVB-induced cytokines secretion (PGE2 and IL-8) was quantified by ELISA, according to the manufacturer's instructions. The ability to attenuate UVB-induced- reduction in viability was measured after 48 hr, by the MTT assay, according to standard operating procedure. Lastly, histological evaluation of collagen and elastin deposits were performed.

The results of these analysis are summarised in Table 11 (viability and inhibition of secretion PGE₂ and IL-8 were determined in comparison to the placebo skin cream). The results demonstrate that the ingredient mixture of the present invention is beneficial and effective in reducing UV induced inflammation when applied as a cream and on human skin transplant, the closest model to human application/clinical trial.

**Table 11**

| Skin cream | Epidermal viability (%) | Inhibition PGE₂ (%) | Inhibition IL-8 (%) |
|---|---|---|---|
| 1 | 131 | 25 | 26 |
| 2 | 138 | 51 | 51 |
| 3 (Placebo) | 100 | 0 | 0 |

Histological evaluation showed that the skin creams according to the invention protected the skin from elastin and collagen reduction following UV exposure and reduced photo-aging effects.

## Claims

1. A topical formulation comprising:
• cannabidiol; and
• omega-3 fatty acid selected from eicosapentaenoic acid and docosahexaenoic acid and combinations thereof.

2. Topical formulation according to claim 1, wherein the formulation contains 0.05-3 wt.% cannabidiol.

3. Topical formulation according to claim 1 or 2, wherein the formulation contains 0.0125 -3 wt.% of the omega-3 fatty acid.

4. Topical formulation according to claim 3, wherein the formulation contains at least 0.0125 wt.% eicosapentaenoic acid.

5. Topical formulation according to any one of claims 1-4, wherein the formulation contains cannabidiol and the omega-3 fatty acid in a weight ratio of 1:8. To 8:1.

6. Topical formulation according to any one of claims 1-5, wherein the formulation additionally contains one or more phenolic acids selected from danshensu, salvianolic acid A, salvianolic acid B and salvianolic acid C.

7. Topical formulation according to claim 6, wherein the formulation contains 0.001-4 wt.% of the one or more phenolic acids.

8. Topical formulation according to claim 6 or 7, wherein the formulation contains an extract of *Salvia miltiorrhiza.*

9. A topical formulation for use in the treatment of ageing or UV-damaged skin, preferably for use in the treatment of photo-aging skin, said treatment comprising topically administrating a formulation comprising cannabidiol and omega-3 fatty acid selected from eicosapentaenoic acid and docosahexaenoic acid and combinations thereof.

10. Topical formulation for use in the treatment according to claim 9, wherein the formulation contains 0.05-3 wt.% cannabidiol.

11. Topical formulation for use in the treatment according to claim 9 or 10, wherein the formulation contains 0.0125-3 wt.% of the omega-3 fatty acid.

12. Topical formulation for use in the treatment according to any one of claims 9-11, wherein the formulation contains cannabidiol and the omega-3 fatty acid in a weight ratio of 1:8. To 8:1.

13. Topical formulation for use in the treatment according to any one of claims 9-12, wherein the formulation additionally contains one or more phenolic acids selected from danshensu, salvianolic acid A, salvianolic acid B and salvianolic acid C, preferably 0.001-4 wt.% of said one or more phenolic acids.

14. A method of preparing a topical formulation according to any one claims 1-8, said method comprising combining an extract of *Cannabis* and an omega-3 oil isolated from a source selected from fish, microalgae, lower fungi, marine bacteria and combinations thereof.

15. Method according to claim 14, wherein the method comprises additionally combining the extract of *Cannabis* and the omega-3 oil with an extract of *Salvia miltiorrhiza.*

## Patentansprüche

1. Topische Formulierung, die Folgendes enthält:
• Cannabidiol; und
• Omega-3-Fettsäure, die aus Eicosapentaensäure und Docosahexaensäure und Kombinationen davon ausgewählt ist.

2. Topische Formulierung nach Anspruch 1, wobei die Formulierung 0,05-3 Gew.-% Cannabidiol enthält.

3. Topische Formulierung nach Anspruch 1 oder 2, wobei die Formulierung 0,0125-3 Gew.-% der Omega-3-Fettsäure enthält.

4. Topische Formulierung nach Anspruch 3, wobei die Formulierung mindestens 0,0125 Gew.-% Eicosapentaensäure enthält.

5. Topische Formulierung nach einem der Ansprüche 1-4, wobei die Formulierung Cannabidiol und die Omega-3-Fettsäure in einem Gewichtsverhältnis von 1:8 bis 8:1 enthält.

6. Topische Formulierung nach einem der Ansprüche 1-5, wobei die Formulierung zusätzlich eine oder mehrere Phenolsäuren enthält, die aus Danshensu, Salvianolsäure A, Salvianolsäure B und Salvianolsäure C ausgewählt sind.

7. Topische Formulierung nach Anspruch 6, wobei die Formulierung 0,001-4 Gew.-% der einen oder mehreren Phenolsäuren enthält.

8. Topische Formulierung nach Anspruch 6 oder 7, wobei die Formulierung einen Extrakt aus *Salvia miltiorrhiza* enthält.

9. Topische Formulierung zur Verwendung bei der Behandlung alternder oder UVgeschädigter Haut, vorzugsweise zur Verwendung bei der Behandlung lichtalternder Haut, wobei die besagte Behandlung das topische Verabreichen einer Formulierung umfasst, die Cannabidiol und Omega-3-Fettsäure enthält, die aus Eicosapentaensäure und Docosahexaensäure und Kombinationen davon ausgewählt ist.

10. Topische Formulierung zur Verwendung bei der Behandlung nach Anspruch 9, wobei die Formulierung 0,05-3 Gew.-% Cannabidiol enthält.

11. Topische Formulierung zur Verwendung bei der Behandlung nach Anspruch 9 oder 10, wobei die Formulierung 0,0125-3 Gew.-% der Omega-3-Fettsäure enthält.

12. Topische Formulierung zur Verwendung bei der Behandlung nach einem der Ansprüche 9-11, wobei die Formulierung Cannabidiol und die Omega-3-Fettsäure in einem Gewichtsverhältnis von 1:8 bis 8:1 enthält.

13. Topische Formulierung zur Verwendung bei der Behandlung nach einem der Ansprüche 9-12, wobei die Formulierung zusätzlich eine oder mehrere Phenolsäuren enthält, die aus Danshensu, Salvianolsäure A, Salvianolsäure B und Salvianolsäure C ausgewählt sind, vorzugsweise 0,001-4 Gew.-% der besagten einen oder mehreren Phenolsäuren.

14. Verfahren zur Herstellung einer topischen Formulierung nach einem der Ansprüche 1-8, wobei das besagte Verfahren das Kombinieren eines Extrakts aus *Cannabis* und eines Omega-3-Öls enthält, das aus einer Quelle isoliert wurde, die aus Fisch, Mikroalgen, niederen Fungi, Meeresbakterien und Kombinationen davon ausgewählt ist.

15. Verfahren nach Anspruch 14, wobei das Verfahren zusätzlich das Kombinieren des Extrakts aus *Cannabis* und des Omega-3-Öls mit einem Extrakt aus *Salvia miltiorrhiza* umfasst.

## Revendications

1. Formulation topique comprenant :
• cannabidiol ; et
• acide gras oméga-3 choisi parmi l'acide eicosapentaénoïque et l'acide docosahexaénoïque et leurs combinaisons.

2. Formulation topique selon la revendication 1, où la formulation contient de 0,05 à 3 % en poids de cannabidiol.

3. Formulation topique selon la revendication 1 ou 2, où la formulation contient de 0,0125 à 3 % en poids de l'acide gras oméga-3.

4. Formulation topique selon la revendication 3, où la formulation contient au moins 0,0125 % en poids d'acide eicosapentaénoïque.

5. Formulation topique selon l'une quelconque des revendications 1 à 4, où la formulation contient du cannabidiol et l'acide gras oméga-3 dans un rapport pondéral de 1:8. à 8:1.

6. Formulation topique selon l'une quelconque des revendications 1 à 5, où la formulation contient en outre un ou plusieurs acides phénoliques choisis parmi le danshensu, l'acide salvianolique A, l'acide salvianolique B et l'acide salvianolique C.

7. Formulation topique selon la revendication 6, où la formulation contient de 0,001 à 4 % en poids de l'un ou plusieurs acides phénoliques.

8. Formulation topique selon la revendication 6 ou 7, où la formulation contient un extrait de *Salvia miltiorrhiza.*

9. Formulation topique pour utilisation dans le traitement de la peau vieillissante ou endommagée par les UV, de préférence pour utilisation dans le traitement de la peau photovieillissante, ledit traitement comprenant l'administration topique d'une formulation comprenant du cannabidiol et un acide gras oméga-3 choisi parmi l'acide eicosapentaénoïque et l'acide docosahexaénoïque et leurs combinaisons.

10. Formulation topique pour utilisation dans le traitement selon la revendication 9, où la formulation contient de 0,05 à 3 % en poids de cannabidiol.

11. Formulation topique pour utilisation dans le traitement selon la revendication 9 ou 10, où la formulation contient de 0,0125 à 3 % en poids de l'acide gras oméga-3.

12. Formulation topique pour utilisation dans le traitement selon l'une quelconque des revendications 9 à 11, où la formulation contient du cannabidiol et l'acide gras oméga-3 dans un rapport pondéral de 1:8 à 8:1.

13. Formulation topique pour utilisation dans le traitement selon l'une quelconque des revendications 9 à 12, où la formulation contient en outre un ou plusieurs acides phénoliques choisis parmi le danshensu, l'acide salvianolique A, l'acide salvianolique B et l'acide salvianolique C, de préférence 0,0014 % en poids desdits un ou plusieurs acides phénoliques.

14. Procédé de préparation d'une formulation topique selon l'une quelconque des revendications 1 à 8, ledit procédé comprenant la combinaison d'un extrait de *Cannabis* et d'une huile oméga-3 isolée à partir d'une source choisie parmi les poissons, les microalgues, les champignons inférieurs, les bactéries marines et leurs combinaisons.

15. Procédé selon la revendication 14, où le procédé comprend en outre la combinaison de l'extrait de *Cannabis* et de l'huile oméga-3 avec un extrait de *Salvia miltiorrhiza.*
